# EUROPEAN PATENT APPLICATION

(11) **EP 4 571 778 A1**
(43) Date of publication of application: **18.06.2025**
(21) Application number: 24172726.2
(22) Date of filing: 26.04.2024
(51) Int. Cl.: G16H 50/20, G16H 10/60, G16H 20/10, G16H 50/30, G16H 50/50, G16H 50/70, G06N 20/00

(54) **ARTIFICIAL INTELLIGENCE SYSTEM AND PREDICTION METHODS THEREOF**

(30) Priority: 12.12.2023 KR 20230180016
(71) Applicant: Pusan National University Industry-University Cooperation Foundation, Busan 46241 (KR)
(72) Inventor: SONG, Gil Tae, 50612 Yangsan-si (KR); KIM, Min Wook, 50612 Yangsan-si (KR); CHOI, Jeong Hoon, 50612 Yangsan-si (KR); KIM, Ju Seong, 50612 Yangsan-si (KR)
(74) Representative: AWA Sweden AB

(57) **Abstract**

An artificial intelligence system comprising, a data module configured to receive personal health record (PHR) data of a patient, perform preprocessing of the PHR data, and generate final data, a model module configured to receive the final data and an instruction and derive a pre-prediction of the patient via at least one artificial intelligence model selected according to the instruction, and a Reliable and Interpretable AI System (RIAS) configured to operate separately from and independently of the data module and the model module, receive the pre-prediction and the final data, and convert the pre-prediction into a final prediction.

## Description

### CROSS-REFERENCE TO RELATED APPLICATION

This application claims priority under 35 U.S.C §119 to Korean Patent Application No. 10-2023-0180016 filed on December 12, 2023, in the Korean Intellectual Property Office, the entire contents of which are hereby incorporated by reference.

### TECHNICAL FIELD

The disclosure relates to an artificial intelligence system and prediction methods thereof. More particularly, the disclosure relates to an artificial intelligence system and prediction methods thereof that can use a variety of data and artificial intelligence models.

### BACKGROUND

PHR (personal health record) data is a record of personal health and can encompass a variety of information. Currently, deriving medical decisions, such as the mortality of patients, by utilizing such PHR data is expanding from the domain of doctors to the domain of artificial intelligence. Medical diagnostic artificial intelligence typically takes the approach of developing a single artificial intelligence model for a certain type of data and drawing a conclusion therefrom. In other words, the approach involves constructing an independent pipeline according to the data and then constructing a new pipeline when the artificial intelligence model is updated.

This approach can have inefficiencies in that even if there are overlapping portions between the respective pipelines, they cannot be utilized and are discarded, and a completely new pipeline must be used. Therefore, studies to eliminate these inefficiencies have remained in existence.

### Prior Art Literature

### Patent Documents

Korean Patent Application Publication No. 10-2023-0140774

### SUMMARY

It is an object of the present disclosure to provide an artificial intelligence system that does not need to be modified even if data and artificial intelligence models change.

In addition, it is another object of the present disclosure to provide a prediction method of an artificial intelligence system that does not need to be modified even if data and artificial intelligence models change.

Further, it is yet another object of the present disclosure to provide a computer program stored on a recording medium that executes a prediction method of an artificial intelligence system that does not need to be modified even if data and artificial intelligence models change.

The objects of the present disclosure are not limited to the objects mentioned above, and other objects and advantages of the present disclosure that have not been mentioned can be understood by the following description and will be more clearly understood by the embodiments of the present disclosure. Further, it will be readily appreciated that the objects and advantages of the present disclosure may be realized by the means set forth in the claims and combinations thereof.

According to some aspects of the disclosure, an artificial intelligence system comprises, a data module configured to receive personal health record (PHR) data of a patient, perform preprocessing of the PHR data, and generate final data, a model module configured to receive the final data and an instruction and derive a pre-prediction of the patient via at least one artificial intelligence model selected according to the instruction, and a Reliable and Interpretable AI System (RIAS) configured to operate separately from and independently of the data module and the model module, receive the pre-prediction and the final data, and convert the pre-prediction into a final prediction.

According to some aspects, the patient comprises a patient with an acute myocardial infarction.

According to some aspects, the final prediction comprises a mortality of the patient after a certain period of time.

According to some aspects, the final prediction comprises a proportion of factors contributing positively to the mortality and a proportion of factors contributing negatively to the mortality.

According to some aspects, the PHR data comprises a mortality after an acute myocardial infarction of the patient or reverse remodeling data after an acute myocardial infarction of the patient.

According to some aspects, the PHR data further comprises data obtained by modifying or manipulating the mortality or the remodeling data.

According to some aspects, the data module comprises, a preprocessing module configured to receive an instruction including a preset dataframe, preprocess the PHR data according to the dataframe, and generate preprocessed data, and an imputation module configured to handle missing values in the preprocessed data and generate final data.

According to some aspects, the imputation module receives the instruction and handles the missing values according to the dataframe.

According to some aspects, the imputation module handles the missing values via at least one of mean imputation, median imputation, mode imputation, regression imputation, K-nearest neighbor imputation, and multiple imputation.

According to some aspects, the instruction comprises information on a predetermined artificial intelligence model.

According to some aspects, the at least one artificial intelligence model can be classified into one type if the at least one artificial intelligence model has a function in common with each other, and the model module objectifies the function in common according to the type.

According to some aspects, the type comprises at least one of GBDT (gradient-boosted decision trees), PyTorch tabular, and Saint.

According to some aspects, the RIAS comprises at least one of, a training module configured to train the artificial intelligence model and tune and evaluate hyperparameters of the artificial intelligence model, a calibrating module configured to correct the pre-prediction and derive a final prediction, a local explanation module configured to perform a local explanation method for a contribution of each feature on the final data, and a counterfactual explanation module configured to perform a counterfactual explanation method on the final data.

According to some aspects, the pre-prediction is a prediction value between 0 and 1, and the calibrating module derives a final prediction by adjusting the prediction value so that the pre-prediction mimics a likelihood of occurrence of an actual event.

According to some aspects of the disclosure, an artificial intelligence system comprises, a memory, and a processor configured to perform calculations in conjunction with the memory, wherein the processor, generates final data by preprocessing PHR data of a patient, derives a pre-prediction by inputting the final data into a selected artificial intelligence model, and derives a final prediction by correcting the pre-prediction.

Aspects of the disclosure are not limited to those mentioned above and other objects and advantages of the disclosure that have not been mentioned can be understood by the following description and will be more clearly understood according to embodiments of the disclosure. In addition, it will be readily understood that the objects and advantages of the disclosure can be realized by the means and combinations thereof set forth in the claims.

The artificial intelligence system and the prediction methods thereof of the present disclosure do not need to modify the pipeline even if the input data changes.

In addition, as there is no need to modify the pipeline even if the artificial intelligence modules change, an efficient pipeline system can be realized.

Further, the contribution of factors contributing to the prediction can be expressed, making it easier to understand and explain the prediction to the patient.

In addition to what is described above, specific effects of the present disclosure will be described together while illustrating the following specific details for carrying out the present disclosure.

### BRIEF DESCRIPTION OF THE DRAWINGS

FIG. 1 is a block diagram for describing an artificial intelligence system in accordance with some embodiments of the present disclosure.
FIG. 2 is a block diagram for describing the PHR data of FIG. 1.
FIG. 3 is a block diagram for describing the instruction in FIG. 1.
FIG. 4 is an example diagram showing a prediction when the beta-blocker of FIG. 1 is not administered, and FIG. 5 is an example diagram showing a prediction when the beta blocker of FIG. 1 is administered.
FIG. 6 is a block diagram for describing the structure of the artificial intelligence system of FIG. 1.
FIG. 7 is a block diagram for describing the structure of the data module of FIG. 6.
FIG. 8 is a block diagram for describing the structure of the RIAS of FIG. 6.
FIG. 9 is a block diagram for describing the model module of FIG. 6.
FIG. 10 is a flowchart for describing a prediction method of an artificial intelligence system in accordance with some embodiments of the present disclosure.
FIG. 11 is a flowchart for describing in detail the step of generating the final data of FIG. 10.
FIG. 12 is a flowchart for describing in detail the step of deriving the pre-prediction of FIG. 10.
FIG. 13 is a diagram for describing the hardware configuration of an artificial intelligence system in accordance with some embodiments of the present disclosure.

### DETAILED DESCRIPTION OF THE PREFERRED EMBODIMENTS

The terms or words used in the disclosure and the claims should not be construed as limited to their ordinary or lexical meanings. They should be construed as the meaning and concept in line with the technical idea of the disclosure based on the principle that the inventor can define the concept of terms or words in order to describe his/her own inventive concept in the best possible way. Further, since the embodiment described herein and the configurations illustrated in the drawings are merely one embodiment in which the disclosure is realized and do not represent all the technical ideas of the disclosure, it should be understood that there may be various equivalents, variations, and applicable examples that can replace them at the time of filing this application.

Although terms such as first, second, A, B, etc. used in the description and the claims may be used to describe various components, the components should not be limited by these terms. These terms are only used to differentiate one component from another. For example, a first component may be referred to as a second component, and similarly, a second component may be referred to as a first component, without departing from the scope of the disclosure. The term 'and/or' includes a combination of a plurality of related listed items or any item of the plurality of related listed items.

The terms used in the description and the claims are merely used to describe particular embodiments and are not intended to limit the disclosure. Singular forms are intended to include plural forms unless the context clearly indicates otherwise. In the application, terms such as "comprise," "comprise," "have," etc. should be understood as not precluding the possibility of existence or addition of features, numbers, steps, operations, components, parts, or combinations thereof described herein.

Unless otherwise defined, the phrases "A, B, or C," "at least one of A, B, or C," or "at least one of A, B, and C" may refer to only A, only B, only C, both A and B, both A and C, both B and C, all of A, B, and C, or any combination thereof.

Unless being defined otherwise, all terms used herein, including technical or scientific terms, have the same meaning as commonly understood by those skilled in the art to which the disclosure pertains.

Terms such as those defined in commonly used dictionaries should be construed as having a meaning consistent with the meaning in the context of the relevant art, and are not to be construed in an ideal or excessively formal sense unless explicitly defined in the application. In addition, each configuration, procedure, process, method, or the like included in each embodiment of the disclosure may be shared to the extent that they are not technically contradictory to each other.

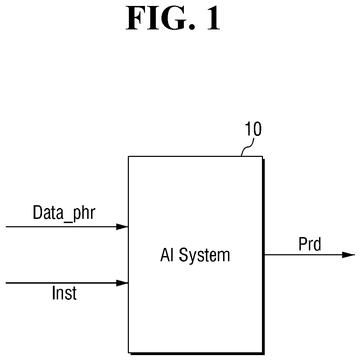

In the following, an artificial intelligence system in accordance with some embodiments of the present disclosure will be described with reference to FIGS. 1 to 9.

FIG. 1 is a block diagram for describing an artificial intelligence system in accordance with some embodiments of the present disclosure.

Referring to FIG. 1, an artificial intelligence system 10 in accordance with some embodiments of the present disclosure may receive PHR data DATA_PHR and an instruction Inst. The artificial intelligence system 10 may generate a prediction Prd internally via the PHR data DATA_PHR and the instruction Inst. The artificial intelligence system 10 may transmit the generated prediction Prd to the outside.

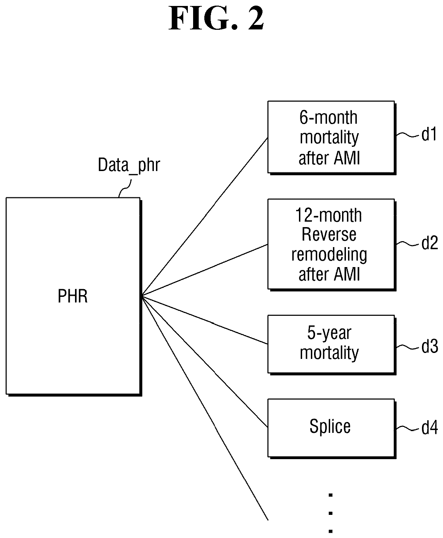

FIG. 2 is a block diagram for describing the PHR data of FIG. 1.

Referring to FIG. 2, the PHR data DATA_PHR may be data on a variety of personal health records of a patient. The PHR data DATA_PHR may be, for example, personal health record data of a patient with an acute myocardial infarction. However, the present embodiment is not limited thereto. The PHR data DATA_PHR may include, for example, at least one of first data d1 on mortality for 6 months from the onset of acute myocardial infarction (AMI), second data d2 on reverse remodeling for 12 months from the onset of acute myocardial infarction, third data d3 on mortality for 5 years from the onset of acute myocardial infarction, and fourth data d4 on the data that has been modified and manipulated from the above data.

In this case, the reverse remodeling is a process in which the heart recovers from damage caused by an acute myocardial infarction, and this may represent a process in which the size, shape, function, etc., of the heart return to normal again. In other words, the reverse remodeling process may include a medical strategy aimed at improving the quality of life of a patient by minimizing damage to cardiac tissue and optimizing cardiac function.

The periods mentioned in the first data d1, second data d2, and third data d3 above are all example periods, and any other periods may be adopted depending on needs and purposes.

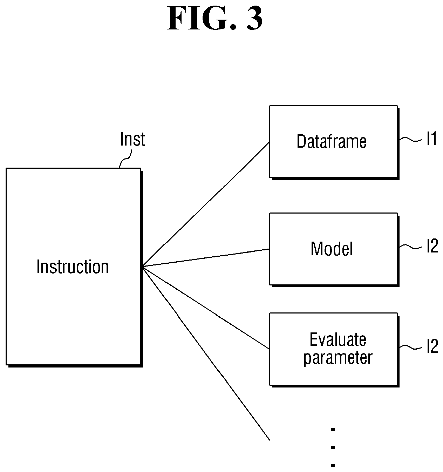

FIG. 3 is a block diagram for describing the instruction in FIG. 1.

Referring to FIGS. 1 to 3, the instruction Inst may include a variety of information necessary for the artificial intelligence system 10 in accordance with the present embodiment to operate.

The instruction Inst may include, for example, at least one of first information I1 on a dataframe, second information I2 on an artificial intelligence model, and third information I3 on evaluation parameters for evaluating the artificial intelligence model.

In this case, the first information I1 may be information on how to preprocess the dataframe of the PHR data DATA_PHR. In other words, the PHR data DATA_PHR may exist in a variety of formats and thus, if it is not converted into a single dataframe, inefficiencies that require a new pipeline system to be constructed newly may occur. The artificial intelligence system 10 in accordance with the present embodiment converts the various PHR data DATA_PHR into a preset dataframe included in the instruction Inst and pass it to the artificial intelligence model, and can thus maximize efficiency as there is no need to construct a new pipeline system.

The second information I2 may be specified information for the artificial intelligence model. In other words, information on which artificial intelligence model is to be used to derive the prediction Prd may be included in the second information I2. Existing prediction systems may result in inefficiencies that require a new pipeline system to be constructed newly in order to introduce latest artificial intelligence model newly. The artificial intelligence system 10 in accordance with the present embodiment can select the artificial intelligence model as a preset model included in the instruction Inst and use functions that overlap with other artificial intelligence models as they are, and can thus enhance efficiency.

The third information 13 may be needed when setting parameters in advance required for the evaluation of the artificial intelligence model. In this case, the third information I3 may be set in advance so that any artificial intelligence model selected out of a variety of artificial intelligence models can be applied.

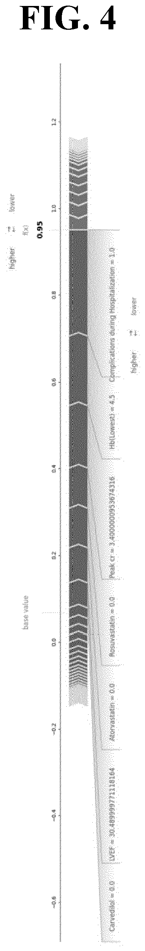

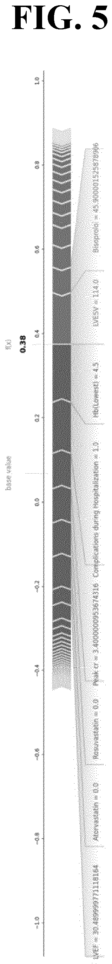

FIG. 4 is an example diagram showing a prediction when the beta-blocker of FIG. 1 is not administered, and FIG. 5 is an example diagram showing a prediction when the beta blocker of FIG. 1 is administered.

Referring to FIGS. 1, 4, and 5, the prediction Prd may represent a probabilistic value such as, for example, the mortality of a patient 12 months after the onset of an acute myocardial infarction. FIG. 4 shows, as an example of the prediction Prd, that the mortality of a patient in a situation where no beta blocker was administered after the onset of an acute myocardial infarction is 95%.

In FIG. 4, the red part may refer to the condition of a patient contributing to the death of the patient, and the blue part may refer to the condition of a patient contributing to the survival of the patient. The prediction Prd of the artificial intelligence system 10 of the present embodiment can provide a contribution to the current prediction Prd value in this way, and thus, analysis and understanding can be made easy.

Referring to FIG. 5, it can be seen that the mortality was reduced to 38% when the beta blocker was administered. In other words, medical professionals responsible for treating patients can get help with diagnosis and prescription by comparing the prediction Prd in FIG. 4 with the prediction Prd in FIG. 5.

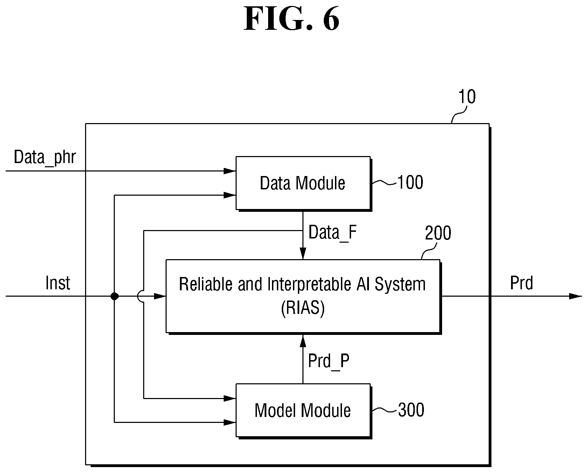

FIG. 6 is a block diagram for describing the structure of the artificial intelligence system of FIG. 1.

Referring to FIG. 6, the artificial intelligence system 10 may include a data module 100, a Reliable and Interpretable AI System (RIAS) 200, and a model module 300.

The data module 100 may receive the PHR data DATA_PHR and the instruction Inst and perform preprocessing. The data module 100 may preprocess the PHR data DATA_PHR according to the instruction Inst and generate final data Data_F. The data module 100 may pass the final data Data_F to the RIAS 200 and the model module 300.

The model module 300 may receive the instruction Inst and the final data Data_F, train an artificial intelligence model, and make predictions. The model module 300 may generate a pre-prediction Prd_P via an artificial intelligence model. The model module 300 may pass the pre-prediction Prd_P to the RIAS 200. At this time, the pre-prediction Prd_P may be a value between 0 and 1.

The RIAS 200 may receive the instruction Inst and the final data Data_F. The RIAS 200 may also receive the pre-prediction Prd_P from the model module 300. The RIAS 200 may derive the pre-prediction Prd_P into a final prediction Prd by using the final data Data_F and the instruction Inst. The RIAS 200 may evaluate and tune the pre-prediction Prd_P.

In this case, the data module 100, the model module 300, and the RIAS 200 may operate as a pipeline system regardless of the type of data and the type of model. In other words, a typical artificial intelligence system generates predictions with an artificial intelligence model determined according to the type of data, and must implement a new pipeline system again instead of the existing pipeline system if the data changes or the artificial intelligence model changes.

On the contrary, in the artificial intelligence system in accordance with the present embodiment, the data module 100 can convert the changed data into the same dataframe and provide it, the model module 300 can perform objectification so as to be able to utilize common functions among a variety of artificial intelligence models, and the RIAS 200 can derive the final prediction Prd independently of data and artificial intelligence models without constructing a new pipeline system.

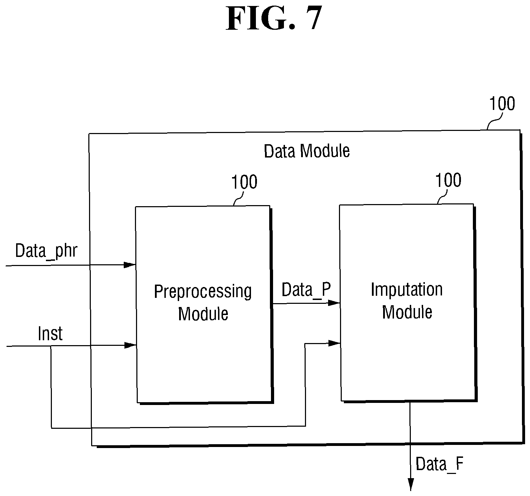

FIG. 7 is a block diagram for describing the structure of the data module of FIG. 6.

Referring to FIG. 7, the data module 100 may include a preprocessing module 110 and an imputation module 120.

The preprocessing module 110 may receive the PHR data DATA_PHR and the instruction Inst. At this time, the instruction Inst may include information on a dataframe. The preprocessing module 110 may preprocess the PHR data DATA_PHR via the information on the dataframe included in the instruction Inst. Accordingly, the preprocessing module 110 may generate preprocessed data Data_P.

The imputation module 120 may receive the preprocessed data Data_P from the preprocessing module 110. Further, the imputation module 120 may receive the instruction Inst and determine a method for handling missing values included in the instruction Inst.

The imputation module 120 may handle missing values in at least one method of, for example, mean imputation, median imputation, mode imputation, regression imputation, K-nearest neighbor imputation, and multiple imputation. In this case, the method selected by the imputation module 120 may be a preset method or a method specified by the instruction Inst. The imputation module 120 may generate final data Data_F by removing the missing values from the preprocessed data Data_P.

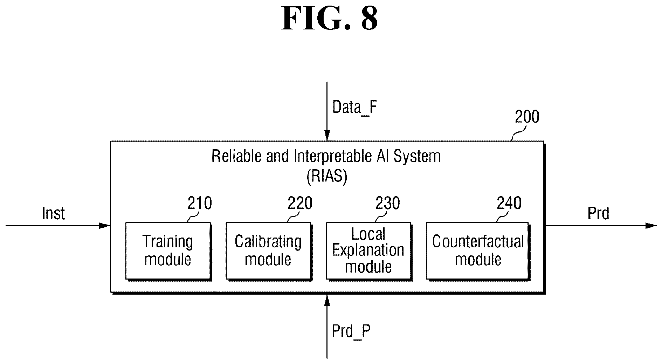

FIG. 8 is a block diagram for describing the structure of the RIAS of FIG. 6.

Referring to FIG. 8, the RIAS 200 may include at least one of a training module 210, a calibrating module 220, a local explanation module 230, and a counterfactual module 240.

The training module 210 may inherit the model of the pre-prediction Prd_P received by the RIAS 200 from the model module 300, and tune, learn, and evaluate hyperparameters of the model. The training module 210 may be able to operate as is even if the artificial intelligence model selected by the model module 300 changes.

The calibrating module 220 may convert the pre-prediction Prd_P into the final prediction Prd. The calibrating module 220 may process the pre-prediction Prd_P to mimic the likelihood of occurrence of an actual event and convert it into a prediction Prd so that a user can accept it as the expected probability of occurrence of the actual event.

The local explanation module 230 may inform how much the features of the final data Data_F utilized in the prediction Prd contributed to the prediction Prd. In other words, the local explanation module 230 may generate the contribution portions in FIGS. 4 and 5.

The counterfactual module 240 may provide an insight into what the user should do to obtain the desired result based on the pre-prediction Prd_P. Accordingly, the prediction Prd may include the insight. For example, an insight that checks mortality with and without beta-blocker administration in FIGS. 4 and 5 and suggests beta-blocker administration may be provided by the counterfactual module 240.

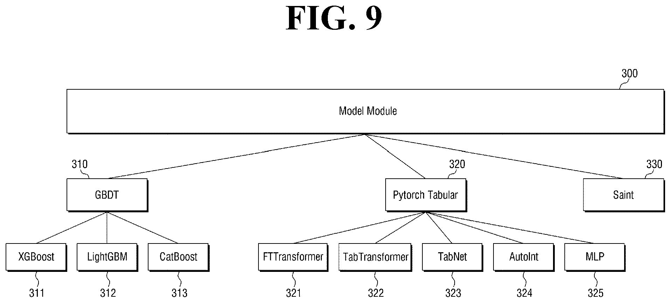

FIG. 9 is a block diagram for describing the model module of FIG. 6.

Referring to FIG. 9, the model module 300 may perform learning and inference by selecting any one of a variety of artificial intelligence models. Each model may be classified into the same type by models that have common functions. Specifically, the artificial intelligence model may include a first type 310, a second type 320, and a third type 330. The number of types is shown as three in FIG. 9, but the present embodiment is not limited thereto.

The first type 310, the second type 320, and the third type 330 may be, for example, gradient-boosted decision trees (GBDT), PyTorch tabular, and Saint, respectively. However, this is merely one example and the present embodiment is not limited thereto.

Accordingly, a first model 311, a second model 312, and a third model 313 belonging to the first type 310 may be XGBoost, LightGBM, and CatBoost, respectively. However, the present embodiment is not limited thereto. A fourth model 321, a fifth model 322, a sixth model 323, a seventh model 324, and an eighth model 325 belonging to the second type 320 may be FTTransformer, TabTransformer, TabNet, AutoInt, and MLP, respectively. However, the present embodiment is not limited thereto.

In this way, the model module 300 can allow pipelines to be shared as much as possible by specifying common objects for artificial intelligence models of the same type having the same function.

The present embodiment may operate independently of data or artificial intelligence models and thus use the same flow no matter what data or model is used. Accordingly, predictions can be derived much more efficiently than existing artificial intelligence systems.

In the following, a prediction method of an artificial intelligence system in accordance with some embodiments of the present disclosure will be described with reference to FIGS. 6 to 8 and 10 to 12. Any description that overlaps with the embodiments described above will be simplified or omitted.

A prediction method of an artificial intelligence system in accordance with some embodiments of the present disclosure may be stored in a recording medium and executed by a computer program. In this case, operations may be performed in organic combination with the artificial intelligence system. However, the present embodiment is not limited thereto.

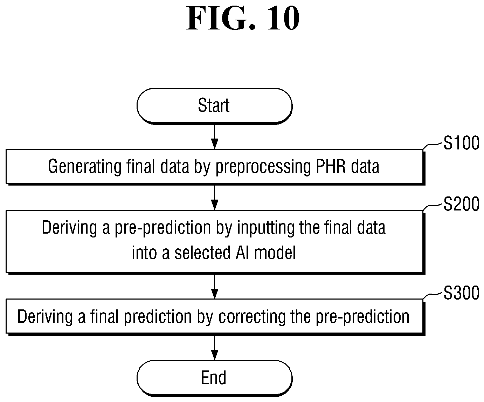

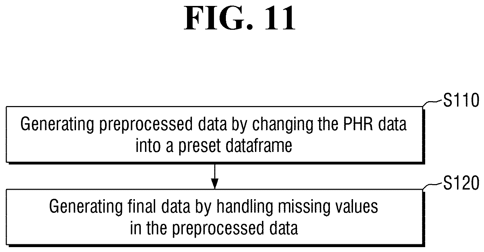

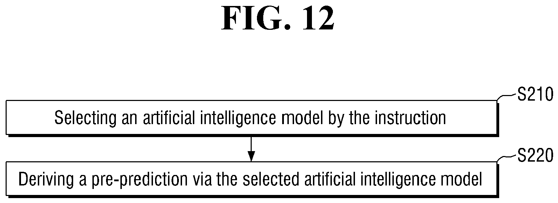

FIG. 10 is a flowchart for describing a prediction method of an artificial intelligence system in accordance with some embodiments of the present disclosure, and FIG. 11 is a flowchart for describing in detail the step of generating the final data of FIG. 10. FIG. 12 is a flowchart for describing in detail the step of deriving the pre-prediction of FIG. 10.

Referring to FIG. 10, final data is generated by preprocessing PHR data (S100).

Referring to FIG. 11 in detail, preprocessed data is generated by changing the PHR data into a preset dataframe (S 110).

Specifically, referring to FIG. 7, the preprocessing module 110 may receive the PHR data DATA_PHR and the instruction Inst. At this time, the instruction Inst may include information on a dataframe. The preprocessing module 110 may preprocess the PHR data DATA_PHR via the information on the dataframe included in the instruction Inst. Accordingly, the preprocessing module 110 may generate preprocessed data Data_P.

Referring again to FIG. 11, final data is generated by handling missing values in the preprocessed data (S120).

Specifically, referring to FIG. 7, the imputation module 120 may receive the preprocessed data Data_P from the preprocessing module 110. Further, the imputation module 120 may receive the instruction Inst and determine a method for handling missing values included in the instruction Inst. The imputation module 120 may generate final data Data_F by removing the missing values from the preprocessed data Data_P.

Referring again to FIG. 10, a pre-prediction is derived by inputting the final data into a selected artificial intelligence model (S200).

Referring to FIG. 12 in detail, an artificial intelligence model is selected by the instruction (S210), and a pre-prediction is derived via the selected artificial intelligence model (S220).

Specifically, referring to FIG. 6, the model module 300 may receive the instruction Inst and the final data Data_F, train the artificial intelligence model, and make predictions. The model module 300 may generate a pre-prediction Prd_P via the artificial intelligence model. The model module 300 may pass the pre-prediction Prd_P to the RIAS 200.

Referring again to FIG. 10, a final prediction is derived by correcting the pre-prediction (S300).

Specifically, referring to FIG. 8, the calibrating module 220 may convert the pre-prediction Prd_P into the final prediction Prd. The calibrating module 220 may process the pre-prediction Prd_P to mimic the likelihood of occurrence of an actual event and convert it into a prediction Prd so that a user can accept it as the expected probability of occurrence of the actual event.

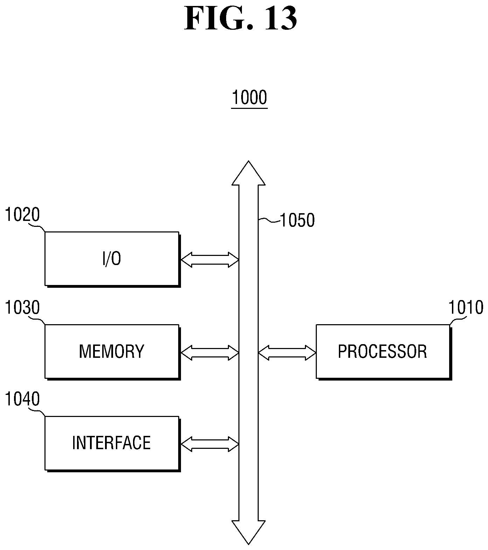

FIG. 13 is a diagram for describing the hardware configuration of an artificial intelligence system in accordance with some embodiments of the present disclosure.

An artificial intelligence system in accordance with some embodiments of the present disclosure may be implemented in an electronic device 1000. The electronic device 1000 may include a processor 1010, an input/output device 1020 (I/O), a memory 1030, an interface 1040, and a bus 1050. The processor 1010, the input/output device 1020, the memory 1030, and/or the interface 1040 may be coupled to each other via the bus 1050. The bus 1050 corresponds to a path along which data travels.

Specifically, the processor 1010 may include at least one of a CPU (central processing unit), an MPU (microprocessor unit), an MCU (microcontroller unit), a GPU (graphics processing unit), a microprocessor, a digital signal processor, a microcontroller, a financial application processor (AP), and logic elements capable of performing similar functions.

The processor 1010 and the memory 1030 of FIG. 13 may be configured as software on the electronic device 1000 or as separate hardware.

The input/output device 1020 may include at least one of a keypad, a keyboard, a touchscreen, and a display device.

The memory 1030 can load data, programs, and the like. In this case, the memory 1030 is an operating memory for improving the operation of the processor 1010, and may include highspeed DRAM and/or SRAM. The memory 1030 may include one or more volatile memory devices such as DDR SDRAM (double data rate static DRAM) and SDR SDRAM (single data rate SDRAM), and/or one or more non-volatile memory devices such as EEPROM (electrically erasable programmable ROM) and flash memory.

The interface 1040 may perform a function of transmitting data to a communication network or receiving data from a communication network. The interface 1040 may be in a wired or wireless form. For example, the interface 1040 may include an antenna, a wired or wireless transceiver, or the like.

A storage can store and hold data, programs, and the like. The storage may include one or more non-volatile memory devices, such as a solid-state drive (SSD), a hard drive, or flash memory. The storage in the present disclosure can store a computer program consisting of instructions for performing the method of providing a digital will service described above.

The input/output device 1020 can be applied to a personal digital assistant (PDA), a portable computer, a web tablet, a wireless phone, a mobile phone, a digital music player, a memory card, or any electronic product that can transmit and/or receive information in a wireless environment.

Further, a quantum circuit simulation device in accordance with embodiments of the present disclosure may be a device formed by connecting a plurality of electronic devices 1000 to each other via a network. In such a case, each module or combination of modules may be implemented as the electronic device 1000. However, the present embodiment is not limited thereto.

Moreover, the quantum circuit simulation device may be implemented in at least one of a workstation, a data center, an internet data center (IDC), a direct attached storage (DAS) system, a storage area network (SAN) system, a network attached storage (NAS) system, and a RAID (redundant array of inexpensive disks, or redundant array of independent disks) system, but the present embodiment is not limited thereto.

Furthermore, the quantum circuit simulation device can transmit data over a network. The network may include a network based on wired Internet technology, wireless Internet technology, and short-distance communication technology. The wired Internet technology may include, for example, at least one of a local area network (LAN) and a wide area network (WAN).

While the inventive concept has been particularly shown and described with reference to exemplary embodiments thereof, it will be understood by those of ordinary skill in the art that various changes in form and details may be made therein without departing from the spirit and scope of the inventive concept as defined by the following claims. It is therefore desired that the embodiments be considered in all respects as illustrative and not restrictive, reference being made to the appended claims rather than the foregoing description to indicate the scope of the disclosure.

## Claims

1. An artificial intelligence system comprising:
a data module configured to receive personal health record (PHR) data of a patient, perform preprocessing of the PHR data, and generate final data;
a model module configured to receive the final data and an instruction and derive a pre-prediction of the patient via at least one artificial intelligence model selected according to the instruction; and
a Reliable and Interpretable AI System (RIAS) configured to operate separately from and independently of the data module and the model module, receive the pre-prediction and the final data, and convert the pre-prediction into a final prediction.

2. The artificial intelligence system of claim 1, wherein the patient comprises a patient with an acute myocardial infarction.

3. The artificial intelligence system of claim 1, wherein the final prediction comprises a mortality of the patient after a certain period of time.

4. The artificial intelligence system of claim 3, wherein the final prediction comprises a proportion of factors contributing positively to the mortality and a proportion of factors contributing negatively to the mortality.

5. The artificial intelligence system of claim 1, wherein the PHR data comprises a mortality after an acute myocardial infarction of the patient or reverse remodeling data after an acute myocardial infarction of the patient.

6. The artificial intelligence system of claim 5, wherein the PHR data further comprises data obtained by modifying or manipulating the mortality or the remodeling data.

7. The artificial intelligence system of claim 1, wherein the data module comprises:
a preprocessing module configured to receive an instruction including a preset dataframe, preprocess the PHR data according to the dataframe, and generate preprocessed data; and
an imputation module configured to handle missing values in the preprocessed data and generate final data.

8. The artificial intelligence system of claim 7, wherein the imputation module receives the instruction and handles the missing values according to the dataframe.

9. The artificial intelligence system of claim 7, wherein the imputation module handles the missing values via at least one of mean imputation, median imputation, mode imputation, regression imputation, K-nearest neighbor imputation, and multiple imputation.

10. The artificial intelligence system of claim 1, wherein the instruction comprises information on a predetermined artificial intelligence model.

11. The artificial intelligence system of claim 10, wherein the at least one artificial intelligence model can be classified into one type if the at least one artificial intelligence model has a function in common with each other, and the model module objectifies the function in common according to the type.

12. The artificial intelligence system of claim 11, wherein the type comprises at least one of GBDT (gradient-boosted decision trees), PyTorch tabular, and Saint.

13. The artificial intelligence system of claim 1, wherein the RIAS comprises at least one of:
a training module configured to train the artificial intelligence model and tune and evaluate hyperparameters of the artificial intelligence model;
a calibrating module configured to correct the pre-prediction and derive a final prediction;
a local explanation module configured to perform a local explanation method for a contribution of each feature on the final data; and
a counterfactual explanation module configured to perform a counterfactual explanation method on the final data.

14. The artificial intelligence system of claim 13, wherein the pre-prediction is a prediction value between 0 and 1, and
the calibrating module derives a final prediction by adjusting the prediction value so that the pre-prediction mimics a likelihood of occurrence of an actual event.

15. An artificial intelligence system comprising:
a memory; and
a processor configured to perform calculations in conjunction with the memory,
wherein the processor:
generates final data by preprocessing PHR data of a patient,
derives a pre-prediction by inputting the final data into a selected artificial intelligence model, and
derives a final prediction by correcting the pre-prediction.
